**Europäisches Patentamt**

**European Patent Office**

**Office européen des brevets**

(11) Publication number: **0 226 639**
**B1**

## (12) EUROPEAN PATENT SPECIFICATION

(45) Date of publication of patent specification: **14.11.90**

(21) Application number: **85902656.9**

(22) Date of filing: **29.05.85**

(86) International application number:
**PCT/JP85/00299**

(87) International publication number:
**WO 86/07093 04.12.86 Gazette 86/26**

(51) Int. Cl.⁵: **C 12 P 21/00, C 07 K 3/12**

## (54) PROCESS FOR PREPARING HETEROGENIC PROTEIN.

(43) Date of publication of application:
**01.07.87 Bulletin 87/27**

(45) Publication of the grant of the patent:
**14.11.90 Bulletin 90/46**

(84) Designated Contracting States:
**BE CH DE FR GB LI NL SE**

(56) References cited:
**EP-A-0 114 506**
**EP-A-0 126 230**
**EP-A-0 140 127**
**EP-A-0 145 390**
**EP-A-0 147 819**
**JP-A-56 154 499**
**JP-A-58 089 196**
**JP-A-58 174 396**

(73) Proprietor: **THE GREEN CROSS CORPORATION**
**3-3, Imabashi 1-chome Chuo-ku**
**Osaka-shi Osaka 541 (JP)**

(72) Inventor: **WATANABE, Hironobu**
**4-7-23-1408, Nankonaka Suminoe-ku**
**Osaka-shi Osaka 559 (JP)**
Inventor: **HIRAMATSU, Ryuji**
**14-1-1008, Kayashimashinwa-cho**
**Neyagawa-shi Osaka 572 (JP)**
Inventor: **TANABE, Yoshizumi**
**4-1-1-507, Nishimakino**
**Hirakata-shi Osaka 573 (JP)**
Inventor: **ISHIKAWA, Hideyuki**
**33-402, Senriokanaka**
**Suita-shi Osaka 565 (JP)**
Inventor: **NAGAI, Masanori**
**10-10, Nishiyamawaki**
**Yawata-shi Kyoto 614 (JP)**
Inventor: **ARIMURA, Hirofumi**
**2-18-1-401, Uenosaka**
**Toyonaka-shi Osaka 560 (JP)**

(74) Representative: **Laredo, Jack Joseph et al**
**Elkington and Fife Beacon House 113 Kingsway**
**London, WC2B 6PP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Technical field

This invention relates to a method of producing heterologous proteins by recovering heterologous proteins from transformants capable of producing heterologous proteins as obtained by gene manipulation techniques.

Background art

Recombinant DNA technology has made it possible to cause expression and production of heterologous proteins in bacteria and other hosts.

For example, many human proteins, including various types ($\alpha$, $\beta$, $\gamma$) of human interferon (hereinafter, HIFN), urokinase and TPA (human tissue plasminogen activating factor) are produced in hosts by transforming the hosts with DNA coding for these proteins and propagating the thus-obtained transformants under conditions suited for the expression of said heterologous proteins. However, heterologous proteins are often insolubilized and precipitate in hosts. In case the desired proteins form such precipitates, the recovery of these encounters the following problems. First, it becomes necessary that those proteins which occur as precipitates in hosts should be separated from host substances and, in addition, solubilized. Second, desired proteins mostly occur in biologically inactive form.

For solving these problems, the prior art has proposed a method comprising treatment with a protein-denaturing agent (Japanese Kokai Tokkyo Kohno 59-161321) and a method comprising treatment with hydrochloric acid-guanidine (USP 4476049). However, these methods are still unsatisfactory with respect to the recovery of desired proteins.

Disclosure of the invention

It is an object of the present invention to provide a method of recovering heterologous proteins from genetically engineered transformants in higher yields by solubilizing the heterologous proteins occurring as precipitates in said transformants to thereby restore the forms in which they are active in bioassays.

The present invention thus provides a method of producing heterologous proteins by cultivating a genetically engineered transformant capable of producing a heterologous protein and recovering the thus-produced heterologous protein, which is characterised in that, a crude suspension of lysed transformant cells with the heterologous protein produced contained therein, are directly treated with a salt solution whereby the heterologous protein is solubilized with at least one ion selected from among the chloride ion, thiocyanate ion and nitrate ion each derived from a dissociable salt.

(a) Starting materials

The starting materials in the practice of the invention are a transformant obtained by genetic engineering techniques and capable of heterologous protein expression, and a host in which the heterologous protein can be produced and accumulated, in particular a bacterial strain. Strains of *Escherichia coli* are preferred examples.

Transformants capable of producing various heterologous proteins can be obtained by the known genetic engineering techniques or modifications thereof. Such techniques are disclosed, for example, in Japanese Patent Application 59-37119 (Applicant: The Green Cross Corporation) relative to a method of producing urokinase, in Japanese Patent Application 59-229864 (Applicant: The Green Cross Corporation) relative to a method of producing IFN-$\gamma$, in Japanese Kokai Tokkyo Koho 58-104887 and Japanese Kokai Tokkyo Koho 59-48082 relative to a method of producing HBsAg and in Japanese Kokai Tokkyo Koho 59-42321 relative to a method of producing TPA.

(b) Heterologous protein recovery from transformant
Removal of transformant cell wall

This is performed, for example, by treating the transformant (cells) with an enzyme digesting cell wall polysaccharides, such as lysozyme. On that occasion, the outer cell wall is removed by suspending the transformant (cells), for example, in a buffer (pH 5—10, preferably pH 7—8) containing the cell wall polysaccharide-digesting enzyme and a chelating agent such as EDTA. Examples of the buffer are 10 mM phosphate buffer (pH 8.0) and 250 mM Tris-hydrochloride buffer (pH 8.0), among others. The transformant (cell) concentration is preferably such that the $OD_{650}$ value amounts to 100—200. The cell wall polysaccharide-digesting enzyme concentration, when the enzyme is lysozyme, for instance, is preferably adjusted to 0.1—1 mg/ml, and the chelating agent concentration to 10—100 mM. The suspension period preferably amounts to 10—60 minutes and the treatment temperature to 0°C±3°C.

Solubilization:

The above suspension treatment is followed by treatment with a salt which, upon dissociation, affords at least one ion selected from among the chloride ion, thiocyanate ion and nitrate ion. Generally, a buffer containing such salt is added. Preferred examples of the salt are alkali metal salts (sodium salts, potassium salts, etc.) and alkaline earth metal salts (calcium salts, magnesium salts, etc.), which are ionically dissociable as mentioned above, and particularly preferred are alkaline earth metal chlorides, alkali metal thiocyanates and alkali metal nitrates. Specifically, magnesium chloride, potassium thiocyanate, sodium thiocyanate and sodium nitrate, among others, are suitable examples. The same buffer as mentioned above is used as the buffer. When the salt is a chloride or nitrate ion-releasing one, the salt is added to a final concentration of 0.15—6 M, preferably 0.25—3 M and, when the salt is a thiocyanate ion-releasing one, to a final concentration of 0.25—1.5 M, preferably 0.3—1.0 M. The treatment is carried out generally under conditions of about 10—60 minutes and about 0°C±3°C. Desired proteins are thus solubilized.

Recovery:

The above-mentioned solubilization treatment is generally followed by recovery of the heterologous proteins. Those techniques which are conventional for protein purification may be used suitably for the recovery. For instance, fractionation based on the solubility difference, such as fractionation with ammonium sulfate, fractionation with ethanol and fractionation with acrinol, and centrifugation are preferable.

The fractionation with ammonium sulfate is carried out by combining a fractionation step using a 10—20% quantity of saturated ammonium sulfate and a fractionation step using a 40—50% quantity of saturated ammonium sulfate.

The centrifugation is carried out at 10,000—25,000 rpm for 10—60 minutes. The sediment is removed and the supernatant is recovered.

Dialysis:

Optionally, the fraction recovered is then dialyzed. On the occasion of dialysis, a buffer (e.g. phosphate buffer) preferably having a pH of 6—8 and a concentration of 0.01—0.2 M is used for salt concentration adjustment. Preferably, the dialysis is performed for a period not shorter than 30 hours.

High-degree purification:

Various heterologous proteins can be purified to a high degree as desired by appropriate means of fractionation selected for the respective heterologous proteins (preferably by affinity chromatography using monoclonal antibodies).

Formulation:

Preparations containing such highly purified heterologous proteins are produced by conventional methods *per se* known and selected depending on the purpose for which the respective preparations are produced. In the case of drug production, the proteins are subjected, if desired, to treatment for removal of microorganisms and for sterilization and then are made up into liquid or dry preparations following investigations as to excipients, stabilizers and solvents.

The production method according to the invention is applicable to the production of any heterologous proteins that can be produced in genetically engineered transformants, such as, for example, interferons (α, β and γ), urokinase, HBsAg and TPA.

The following working examples and experimental examples are given for the purpose of illustrating the present invention in more detail.

Example 1

The *Escherichia coli* strain W3110 carrying the plasmid PYS61 (Japanese Patent Application 59-229864) with a human interferon-γ polypeptide-encoding DNA fragment inserted therein was cultivated overnight at 37°C in LB+glucose medium (prepared by dissolving 0.63 g of Tris base, 10 g of polypeptone, 5 g of yeast extract, 5 g of NaCl, 1 g of glucose and 20 mg of ampicillin in water to make one liter) and a 2-ml portion of the culture was used to inoculate 100 ml of KM-9 medium (prepared by dissolving 120 g of $Na_2HPO_4$, 60 g of $NaH_2PO_4$, 20 g of NaCl, 300 g of casamino acids and 50 g of yeast extract in water to make 10 liters). After cultivation at 37°C for 1 hour, indoleacrylic acid, a tryptophan gene inducer, was added to 40 μg/ml and, then, cultivation was further continued for 7—10 hours. Cells were harvested by centrifugation at 4,000 r.p.m. for 20 minutes and washed with 30 mM NaCl-50 mM Tris-hydrochloride buffer (pH 8.0). The cells thus washed were suspended in a lysing buffer [30 mM NaCl, 50 mM EDTA, 250 mM Tris-hydrochloride (pH 8.0), 10 μg/ml PMSF (phenylmethylsulfonyl fluoride), 1 mg/ml BSA (bovine serum albumin), 0.25 mg/ml lysozyme] to a cell concentration of $OD_{650}=160$, and the suspension was allowed to stand at 0°C for 30 minutes. Then, an equal volume of a buffer having the same composition as the above lysing buffer except that it was free of lysozyme but contained sodium nitrite in a concentration of 2 M was added, and the mixture was allowed to stand at 0°C for 30 minutes. The subsequent centrifugation at 18,000 r.p.m. for 30 minutes gave a supernatant, which was dialyzed against 0.1 M $KH_2PO_4$ (pH 7.0) used as the dialyzing solution and then assayed for interferon-γ (IFN-γ) by the cpe inhibition method [Havell, E. A. and Vilcek, J., Antimicrob. Agents Chemother., vol. 2, pages 476—484 (1972)] [using, howver, Sindbis virus as the virus and human amnion-derived FL3-1 cells as the animal cells]. The results are shown in Table 1. In the control run, the lysozyme treatment was followed by two repeated freezing-thawing cycles and then by assaying.

TABLE 1

| | IFN-γ (IU/l) |
|---|---|
| Control | $1.8 \times 10^8$ |
| Example 1 (NaNO₃) | $6.0 \times 10^8$ |

Example 2

The same bacterial strain as used in Example 1 was cultivated and cells were harvested and washed in the same manner as in Example 1. The cells (corresponding to 1.8 liters of culture broth) were divided into two equal portions and each portion was suspended in the lysing buffer in a cell concentration corresponding to $OD_{650}=160$, and the suspension was allowed to stand at 0°C for 30 minutes. Then, an equal volume of a buffer having the same composition as the above lysing buffer except that it was free of lysozyme and contained magnesium chloride in a concentration of 1 M was added and the mixture was allowed to stand at 0°C for 45 minutes. In the control run, the lysozyme treatment was followed by two repeated freezing-thawing cycles. The subsequent 1-hour centrifugation at 10,000 r.p.m. gave a supernatant, which was assayed for IFN-γ by the cpe inhibition method. The results thus obtained are shown in Table 2.

TABLE 2

| | IFN-γ (IU/l) |
|---|---|
| Control | $2.9 \times 10^8$ |
| Example 2 (MgCl₂) | $9.3 \times 10^8$ |

Example 3

The *Escherichia coli* HB101 strain carrying the plasmid PYS61 (Japanese Patent Application 59-229864) was cultivated and cells were harvested and washed in the same manner as in Example 2. The cells (corresponding to 1.8 liters of culture broth) were divided into two equal portions and IFN-γ was extracted in the same manner as in Example 2. The results thus obtained are shown in Table 3.

TABLE 3

| | IFN-γ (IU/l) |
|---|---|
| Control | $3.6 \times 10^8$ |
| Example 3 (MgCl₂) | $9.4 \times 10^8$ |

Example 4

The TPA-producing *Escherichia coli* HB101 strain was cultivated and cells were harvested and washed in the same manner as in Example 1. The cells were suspended in the lysing buffer in a cell concentration of $OD_{650}=160$, and the suspension was allowed to stand at 0°C for 30 minutes. Then, an equal volume of a buffer having the same composition as the above lysing buffer except that it was free of lysozyme and contained sodium nitrate in a concentration of 2 M was added. The mixture was allowed to stand at 0°C for 45 minutes and then centrifuged at 18,000 r.p.m. for 30 minutes to give a supernatant. β-Mercaptoethanol was added to the supernatant to a final concentration of 30 mM and the mixture was allowed to stand at room temperature for 2 hours. The lysate was dialyzed against 500 volumes of dialyzing solution A [30 mM NaCl, 10 mM EDTA, 50 mM Tris-hydrochloride (pH 8.0), 1 mM reduced form glutathione, 10% glycerol]. Reduced form glutathione and oxidized form glutathione were added to the dialyzate in final concentrations of 10 mM and 1 mM, respectively, and the resultant solution was allowed to stand overnight at room temperature and then finally dialyzed against 500 volumes of dialyzing solution B [30 mM NaCl, 10 mM EDTA, 50 mM Tris-hydrochloride, 0.1 mM reduced form glutathione]. The dialyzate was assayed for PA activity (plasminogen activator activity) by the fibrin plate method. The results thus obtained are shown in Table 4. As a result, an about 50-fold increase in PA activity was observed as compared with the control (two repeated freezing-thawing cycles following lysozyme treatment).

TABLE 4

|  | TPA (U/ml) |
|---|---|
| Control | 0.5 |
| Example 4* (NaNO$_3$) | 1.8 |
| Example 4** | 27.0 |

*: The PA activity was measured after dialysis following sodium nitrate treatment.

**: The PA activity was measured after sodium nitrate treatment, β-mercaptoethanol treatment and oxidation treatment in the GSH/GSSG-containing buffer. GSH means reduced form glutathione and GSSG oxidized form glutathione.

Experimental Example 1

The effects of various salts added were comparatively examined by carrying out IFN-γ extraction and recovery from the transformant in the same manner as in Example 1. The results obtained are summarized in Table 5. Each additive was added in lieu of sodium nitrate in Example 1, and, otherwise, the whole procedure was the same as in Example 1. The additives, addition levels and IFN-γ activity data are shown in Table 5.

TABLE 5

| Additive | Final concentration | IFN-γ (IU/l) |
|---|---|---|
| Control |  | $1.8 \times 10^8$ |
| MgCl$_2$ | 0.1 | $1.2 \times 10^8$ |
|  | 0.25 | $5.5 \times 10^8$ |
|  | 0.5 | $9.4 \times 10^8$ |
|  | 2 | $5.8 \times 10^8$ |
|  | 5 | $4.8 \times 10^6$ |
| NaNO$_3$ | 0.1 | $1.0 \times 10^8$ |
|  | 0.25 | $4.3 \times 10^8$ |
|  | 0.5 | $4.4 \times 10^8$ |
|  | 2 | $6.0 \times 10^8$ |
|  | 5 | $5.0 \times 10^6$ |
| KSCN | 0.2 | $8.3 \times 10^7$ |
|  | 0.3 | $3.5 \times 10^8$ |
|  | 0.5 | $7.0 \times 10^8$ |
|  | 1.5 | $5.0 \times 10^8$ |
|  | 2 | $7.4 \times 10^7$ |
| Urea | 0.5 | $1.4 \times 10^8$ |
|  | 2 | $1.8 \times 10^8$ |
| Guanidine hydrochloride | 0.5 | $2.3 \times 10^8$ |
|  | 2 | $1.2 \times 10^8$ |

The present invention thus provided has achieved about 4-fold increase in yield as compared with the conventional extraction/recovery method. As a result, the present invention makes it possible to practice, on a commercial level, those gene recombination techniques which use *Escherichia coli* and the like as hosts and which have been so far unsuccessful in achieving satisfactory production efficiency for reasons of intracellular product accumulation.

## Claims

1. A method of producing heterologous proteins by cultivating a genetically engineered transformant capable of producing a heterologous protein and recovering the thus-produced heterologus protein, which is characterized in that, a crude suspension of lysed transformant cells with the heterologous protien produced contained therein, are directly treated with a salt solution whereby the heterologous protein is solubilized with at least one ion selected from among the chloride ion, thiocyanate ion and nitrate ion each derived from a dissociable salt.

2. A method of producing heterologous proteins as claimed in Claim 1, wherein the salt is an alkali metal salt or an alkaline earth metal salt.

3. A method of producing heterologous proteins as claimed in Claim 1, wherein the salt is at least one salt selected from among alkaline earth metal chlorides, alkali metal thiocyanates and alkali metal nitrates.

4. A method of producing heterologous proteins as claimed in Claim 1, wherein the salt is at least one salt selected from among magnesium chloride, potassium thiocyanate, sodium thiocyanate and sodium nitrate.

5. A method of producing heterologous proteins as claimed in Claim 1, wherein a chloride ion-releasing dissociable salt and/or a nitrate ion-releasing dissociable salt is used in a concentration of 0.25—2 M.

6. A method of producing heterologous proteins as claimed in Claim 1, wherein a thiocyanate ion-releasing dissociable salt is used in a concentration of 0.3—1.5 M.

## Patentansprüche

1. Verfahren zur Herstellung heterologer Proteine durch Kultivieren eines gentechnologisch (durch "genetic engineering") hergestellten Transformanten, der zur Bildung eines heterologen Proteins befähigt ist, und Isolieren des so hergestellten heterologen Proteins, dadurch gekennzeichnet, daß eine rohe Suspension lysierter Transformanten-Zellen mit dem darin enthaltenen erzeugten Protein direkt mit einer Salz-Lösung behandelt werden, wodurch das heterologe Protein mit wenigstens einem Ion solubilisiert wird, das aus dem Chlorid-Ion, Thiocyanat-Ion und Nitrat-Ion ausgewählt ist, die jeweils aus einem dissoziierbaren Salz stammen.

2. Verfahren zur Herstellung heterologer Proteine nach Anspruch 1, worin das Salz ein Alkalimetall-Salz oder ein Erdalkalimetall-Salz ist.

3. Verfahren zur Herstellung heterologer Proteine nach Anspruch 1, worin das Salz wenigstens ein Salz ist, das aus Erdalkalimetallchloriden, Alkalimetallthiocyanaten und Alkalimetallnitraten ausgewählt ist.

4. Verfahren zur Herstellung heterologer Proteine nach Anspruch 1, worin das Salz wenigstens ein Salz ist, das aus Magnesiumchlorid, Kaliumthiocyanat, Natriumthiocyanat und Natriumnitrat ausgewählt ist.

5. Verfahren zur Herstellung heterologer Proteine nach Anspruch 1, worin ein Chlorid-Ionen freisetzendes dissoziierbares Salz und/oder ein Nitrat-Ionen freisetzendes dissoziierbares Salz in einer Konzentration von 0,25 M bis 2 M verwendet wird.

6. Verfahren zur Herstellung heterologer Proteine nach Anspruch 1, worin ein Thiocyanat-Ionen freisetzendes dissoziierbares Salz in einer Konzentration von 0,3 M bis 1,5 M verwendet wird.

## Revendications

1. Procédé de production de protéines hétérologues en cultivant un transformant obtenu à l'aide du génie génétique, capable de produire une protéine hétérologue et en récupérant la protéine hétérologue ainsi préparée, caractérisé en ce qu'une suspension brute de transformants cellulaires lysés contenant la protéine hétérologue produite est traitée directement par une solution saline, la protéine hétérologue étant solubilisée avec au moins un ion choisi parmi l'ion chlorure, l'ion thiocyanate et l'ion nitrate, chacun dérivé d'un sel dissociable.

2. Procédé de production de protéines hétérologues selon la revendication 1, dans lequel le sel est un sel de métal alcalin ou un sel de métal alcalino-terreux.

3. Procédé de production de protéines hétérologues selon la revendication 1, dans lequel le sel est au moins un sel choisi parmi les chlorures de métaux alcalino-terreux, les thiocyanates de métaux alcalins et les nitrates de métaux alcalins.

4. Procédé de production de protéines hétérologues selon la revendication 1, dans lequel le sel est au moins un sel choisi parmi le chlorure de magnésium, le thiocyanate de potassium, le thiocyanate de sodium et le nitrate de sodium.

5. Procédé de production de protéines hétérologues selon la revendication 1, dans lequel un sel dissociable libérant l'ion chlorure et/ou un sel dissociable libérant l'ion nitrate est utilisée à une concentration de 0,25—2 M.

6. Procédé de production de protéines hétérologues selon la revendication 1, dans lequel un sel dissociable libérant l'ion thiocyanate est utilisé à une concentration de 0,3—1,5 M.